(19) Europäisches Patentamt
European Patent Office
Office européen des brevets

(11) Publication number: **0 601 661 A2**

## (12) EUROPEAN PATENT APPLICATION

(21) Application number: **93203412.7**

(22) Date of filing: **04.12.93**

(51) Int. Cl.5: **C08F 12/26**, C08F 220/34, G02B 1/04, C07C 271/12

(30) Priority: **11.12.92 IT MI922813**

(43) Date of publication of application: **15.06.94 Bulletin 94/24**

(84) Designated Contracting States: **AT BE CH DE DK ES FR GB GR IE IT LI LU MC NL PT SE**

(71) Applicant: **ENICHEM SYNTHESIS S.p.A.**
**Via Ruggero Settimo 55**
**I-90139 Palermo(IT)**

(72) Inventor: **Greco, Alberto**
**Via dei Mughetti,2**
**20070 Dresano (Milano)(IT)**
Inventor: **Girelli, Daniele**
**Via Francesco d'Ovido,3**
**20131 Milano(IT)**

(74) Representative: **Lotti, Giorgio et al**
**c/o Ing. Barzanò & Zanardo Milano S.p.A.**
**Corso Vittorio Emanuele II, 61**
**I-10128 Torino (IT)**

# (54) Organic glasses with a high refractive index.

(57) Liquid compositions which can be polymerized thermally or via radicals, basically composed of urethanic compounds containing unsaturations of the styrenic or (meth)acrylic type, selected from those having general formulae (I) or (II):

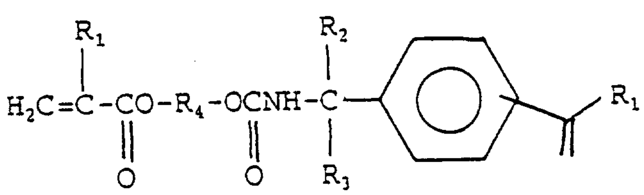

(I)

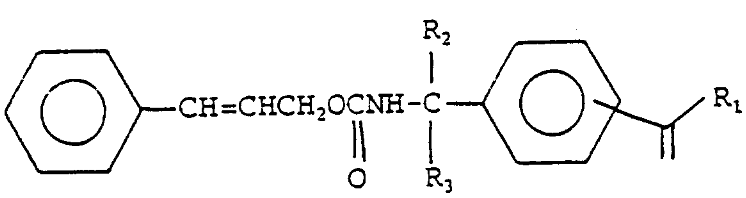

(II)

wherein:

- $R_1$, $R_2$ and $R_3$, the same or different, represent a hydrogen atom or a methyl;
- $R_4$ represents a $C_2$-$C_{10}$ (iso)alkylic residue of at least one diol;

and organic glasses with a high refractive index obtained by radicalic cross-linking of the above compositions.

EP 0 601 661 A2

The present invention relates to liquid compositions, basically composed of urethanic compounds, which can be cross-linked into organic glasses having a high refractive index.

More specifically, the present invention relates to liquid compositions which can be polymerized thermally or via radicals, basically composed of urethanic compounds containing unsaturations of the styrenic or (meth)acrylic type and organic glasses with a high refractive index obtained by the radicalic cross-linking of the above compositions.

The present invention also relates to a procedure for the preparation of the urethanic compounds which form the above liquid compositions.

Organic glasses obtained by the cross-linking of organic compositions based on acrylic, polystyrenic resins or on polycarbonates or their mixtures, are already known in the art.
These glasses are mainly used in the production of optical items and are an alternative to inorganic glasses in that they are easier to obtain, they have a variety of different properties and can be used for various purposes owing to the wide range of polymeric compositions available and their low specific weight.

Among the particular properties of organic glasses are their shock resistance, lack of fragility and processability of the resins from which they derive.

The development of technology in this field is such that more and more sophisticated materials without defects are being obtained and at the same time are required.

Italian patent application IT 21327 A/90 describes organic glasses composed of products of the radicalic polymerization of poly(allylcarbonates) of polyols which are used in the manufacture of optical items. However, the refractive index of this organic glasses is rather low and they are not very suitable for the production of lenses having high diopters and other products for which this characteristic is fundamental and indispensable.

Other properties which, together with a high refractive index, are mainly required from organic glasses, are their transparency, hardness and processability. The production of organic glasses having these properties is one of the objectives which has not yet been satisfactorily resolved in the art.

Various polymeric compositions suitable for the production of organic glasses with a high $n_D^{20}$ refractive index, higher than the minimum value of 1.50, have been proposed in the last ten years; many of these are based on urethanic resins containing unsaturations which can be cross-linked to optical glasses.

Japanese patent application JP 60-11513 discloses (meth)acrylate urethanic resins, diluted in styrenic compounds having the following structure:

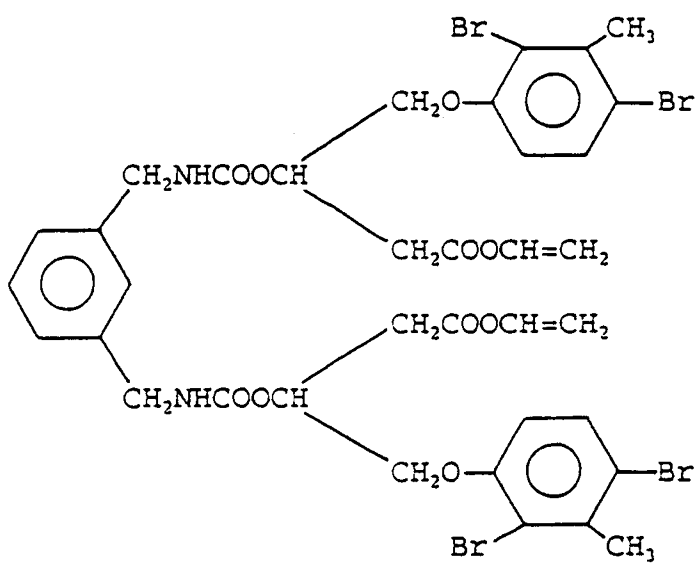

These resins have a refractive index, measured at 20 ° C, higher than 1.55 ($n_D^{20}$ > 1.55), but are difficult to synthesize with suitable characteristics of optical purity in that they are difficult to purify owing to their complexity; in addition, for the same reason, the above resins are not perfectly colourless as is required for the uses for which they are destined.

Japanese patent application JP 60-249101 discloses acrylate urethanic resins, diluted in styrenic compounds, having the following structural formula:

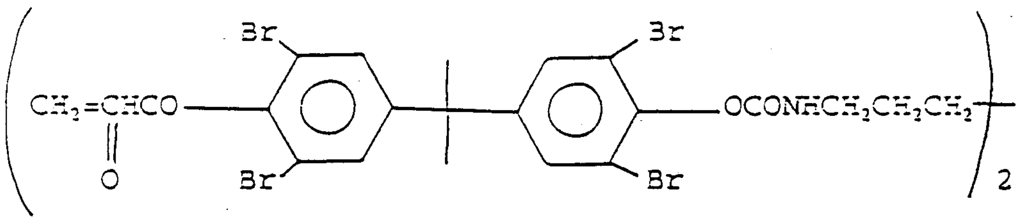

However, also these resins, although having a high refractive index, have the disadvantage of being obtained by reaction in a reactive diluent, of hexamethylendiisocyanate with the monoacrylate of bisphenol-A, in a bicomponent system generally not accepted by the manufacturing industry.

European patent 441.383 describes an acrylate urethanic resin having the following structural formula:

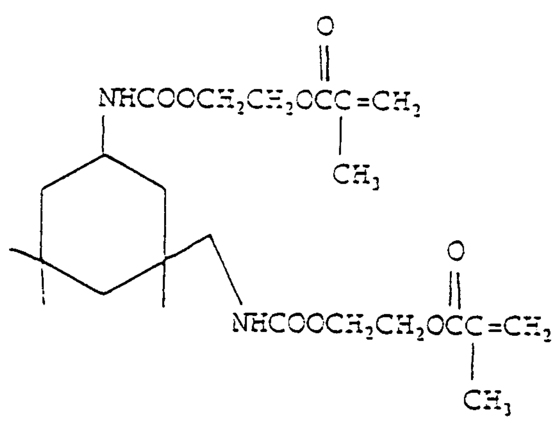

This resin, mixed with dimethacrylates and diluted to 40% by weight in methacrylates, produces, after cross-linking, organic glasses with a $n_D^{20}$ refractive index of about 1.54. The processability of the above resins is good but, in spite of this, the systems are complex with a formula which is not well defined in that structures having a higher molecular weight are present (dimers and trimers) and, as indicated, the refractive index is unsatisfactory.

The Applicant has now found new liquid compositions basically composed of urethanic compounds, capable of producing by cross-linking organic glasses having a high refractive index, $n_D^{20}$ of between 1.54 and 1.60, and of overcoming the disadvantages of the known art.

The main advantage of using the urethanic compounds described below, either alone or mixed with each other, compared to those of the known art, mainly lies in the fact that these compounds are liquid at room temperature with a relatively low viscosity, which allows them to be used in their pure state or with varying quantities of the reactive diluent or reactive diluents specified below or as diluents with respect to other reactive compounds containing unsaturations.

A second advantage consists in the fact that these urethanic compounds can be easily obtained by the reaction of an isocyanate with a hydroxyalkyl(meth)acrylate or a cinnamic alcohol.

The present invention therefore relates to liquid compositions which can be polymerized thermally or via radicals, basically composed of urethanic compounds containing unsaturations of the styrenic or (meth)-acrylic type, selected from those having the following general formulae (I) or (II):

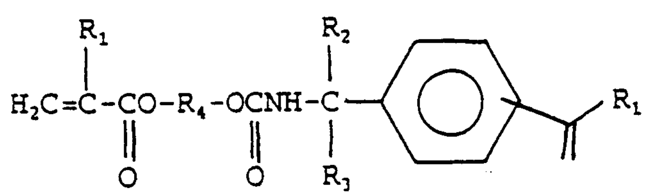

(I)

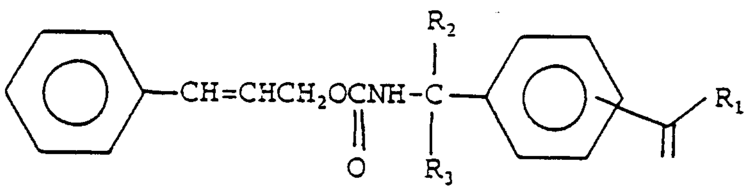 (II)

wherein:

- $R_1$, $R_2$ and $R_3$, the same or different, represent a hydrogen atom or a methyl;
- $R_4$ represents a $C_2$-$C_{10}$ (iso)alkylic residue of at least one diol.

Preferred urethanic compounds having general formula (I) or (II) in accordance with the present invention, are those wherein $R_4$ represents an alkyl residue of a diol having one of the following formulae:

$-CH_2CH_2-$; $-CH_2CHCH_3$;

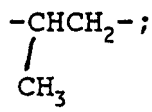

$-CH_2CH_2CH_2CH_2-$.

The urethanic compounds having general formula (I) or (II) can be used either alone or mixed with each other, or can be mixed with one or more reactive diluents of the acrylate and/or methacrylate and/or styrenic type, or mixed with reactive compounds containing unsaturated groups.

The urethanic compounds having general formula (I) or (II) can be mixed with each other in all proportions.

The reactive diluents of the acrylate and/or methacrylate and/or styrenic type with which the urethanic compounds having formula (I) or (II), as such or mixed with each other, are possibly mixed, can be mono, bi- or polyfunctional in that they contain one or more unsaturations. These diluents are selected from those belonging to the groups of acrylic, methacrylic and styrenic compounds which have a high refractive index, low viscosity and good optical properties.

Examples, which do not limit the purposes of the present invention, of reactive diluents of the acrylate and/or methacrylate type are: hexandiol-diacrylate, benzyl-methacrylate, phenyl-methacrylate, phenoxyethyl-methacrylate, cyclohexyl-methacrylate; and also:

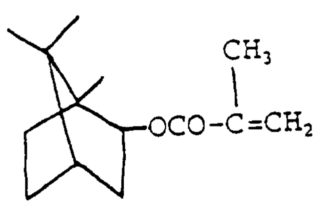

isobornyl-methacrylate;

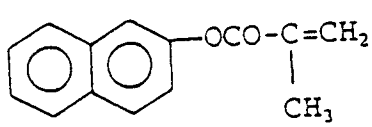

2-naphthyl-methacrylate;

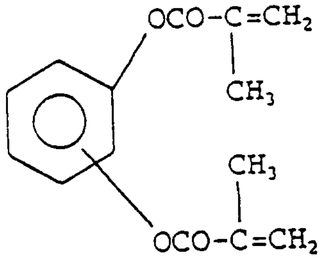

(o, m, p)-phenylene-dimethacrylate;

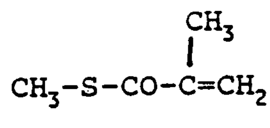

methylthio-methacrylate;

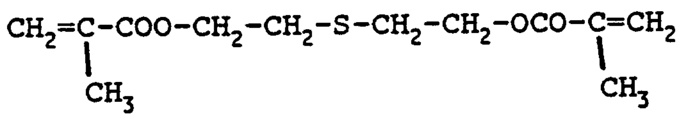

thiodiethylen-dimethacrylate;

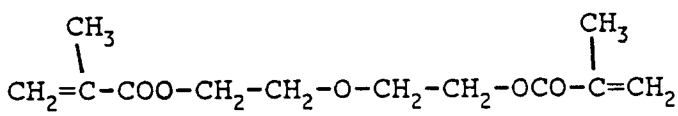

diethylenglycol-dimethacrylate;
and the corresponding acrylates.

Examples of reactive diluents of the styrenic type, are: m-divinylbenzene, m-ethylvinylbenzene, styrene, α-methylstyrene, (o, m, p)-chlorostyrene, (o, m, p)-bromostyrene, 2-isopropenyl-naphthalene.

The above reactive diluents are preferably added to the urethanic compounds having formula (I) or (II), as such or to their mixture, when their synthesis has been completed.

The quantity of reactive diluents of the acrylate and/or methacrylate and/or styrenic type with which the liquid compositions of the present invention are possibly diluted, is such that the weight ratio urethanic compounds/reactive diluent is between 99:1 and 45:55.

The reactive compounds containing unsaturated groups with which the liquid compositions composed of urethanic compounds having formula (I) or (II), as such or mixed with each other, are possibly mixed, can be either mono- or b- or poly-functional in that they contain one or two or more unsaturations.

Unlimiting examples for the purposes of the present invention of reactive compounds containing unsaturated groups are:

a) styrene-urethanic resins as described in Italian patent application MI 92 A 001276 filed by the Applicant having the following structural formula:

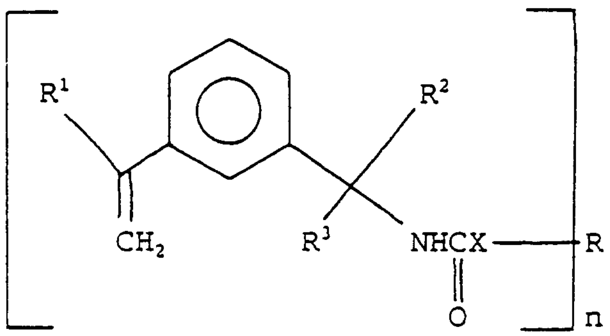

wherein:

- R' represents an alkyl or cyclo-alkyl residue of a glycol, polyol, polythiol, dimercaptan, containing from 2 to 19 carbon atoms and 1 or 2 sulphur or oxygen atoms;
- $R^1$, $R^2$ and $R^3$, the same or different, represent a hydrogen atom or methyl;
- X represents an oxygen or sulphur atom;
- n is 2 or 3;

b) bis(alkyleneoxyphenyl)diacrylates or dimethacrylates, as described in patents GB 2.076.836 and DE 4.010.783, having the following general formula:

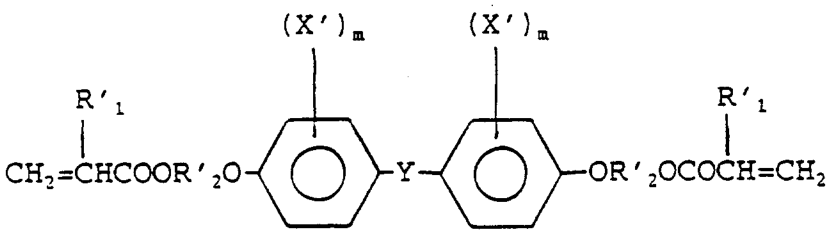

wherein:

- $R'_1$ represents a hydrogen atom or a methyl;
- $R'_2$ represents one of the following groups: $-CH_2CH_2-$;

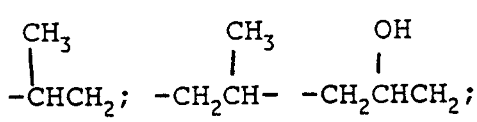

- Y represents an oxygen atom or one of the following groups:
  $-SO_2-$; $-CH_2-$;

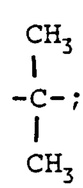

- X' represents a chlorine or bromine atom;
- m is 0, 1 or 2.

The liquid compositions of the present invention also include radicalic polymerization initiators (peroxides or azocompounds) and, optionally, other additives such as stabilizers, bleaching agents, dyes, detaching agents, etc.

The radicalic polymerization initiators are composed of peroxides belonging to the groups of peresters such as t-butylperoxy-2-ethylhexanoate, percarbonates such as dicyclohexylpercabonate or diacylperoxides

such as dibenzoylperoxide. They are added to the liquid composition in a proportion of between 0.01% and 5% by weight, preferably between 0.1% and 3% by weight.

As an alternative to peroxides diazoderivatives can be used, such as, for example, asobisisobutyrronitrile, asobisisovaleronitrile, etc. The percentage of azocompound, when used, varies from 0.001% to 0.5% by weight, preferably between 0.01% and 0.2% by weight.

Other additives can also be added to the liquid compositions of the present invention, as specified above, such as, for example, dyes, U.V. absorbers, etc. Their proportion however is relatively small and does not exceed 1% by weight with respect to the composition which is therefore composed of the following main constituents:

- urethanic compounds having formula (I) or (II), as such or mixed with each other;
- reactive diluents or reactive compounds containing unsaturated groups;
- polymerization initiators;
- other optional components such as dyes, U.V. absorbers, etc.

The present invention also relates to a procedure for the preparation of the urethanic compounds having formula (I) or (II).

A procedure for the preparation of urethanic compounds having formula (I) or (II) includes the reaction of a mole of an isocyanate having formula (III):

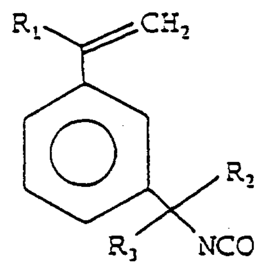

(III)

wherein $R_1$, $R_2$ and $R_3$ have the meaning specified above, with a mole of a hydroxalkyl(meth)acrylate and/or a cinnamic alcohol corresponding to structural formulae (IV) and (V):

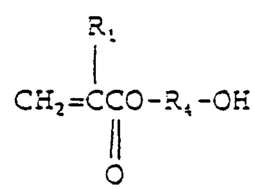

(IV)

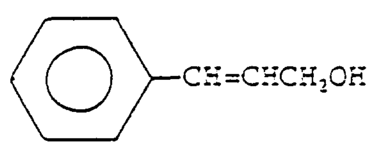

(V)

wherein $R_1$ and $R_4$ have the meaning specified above.

This reaction is generally carried out over a period ranging from 2 to 24 hours, at a temperature of between 20°C and 120°C, adding the hydroxyalkyl(meth)acrylate having formula (IV) or cinnamic alcohol having formula (V) to the isocyanate having formula (III) or viceversa.

The synthesis reaction of the urethanic compounds having formula (I) or (II), can be accelerated by adding the usual catalysts normally used in the synthesis of urethanes (Saunders and Frisch: "Polyurethanes Chemistry and Technology", Interscience, New York, 1964) such as, for example, organometallic derivatives of tin (tin dibutyl-dilaurate) and tertiary amines or their combinations. The quantity of these catalysts generally ranges from 0.01% to 1% by weight with respect to the total quantity of the two reagents: isocyanate having formula (III) + hydroxyalkyl(meth)acrylate having formula (IV) or cinnamic alcohol having formula (V).

Radicalic polymerization inhibitors can be added to the reaction medium to avoid the polymerization of the styenic part of the isocyanate having formula (III). The quantity of these inhibitors is very small and is

between 0.001% and 1% by weight with respect to the total quantity of the two reagents.

The formation reaction of urethane is exothermic and it is therefore necessary to control the temperature during the addition of the hydroxyalkyl(meth)acrylate or cinnamic alcohol to the isocyanate and viceversa. The preferred temperature range is between 50 °C and 70 °C.

The reaction can be carried out in the presence of reactive diluents or part of these even if, as specified above, the latter are preferably added to the urethanic compound when its synthesis has been completed.

The present invention also relates to organic glasses having a high refractive index obtained by the radicalic cross-linking of the above compositions.

For this purpose, the liquid compositions of the present invention are poured into a solid mould, generally made of glasses, whose shape and dimensions vary according to the desired form of the end-product.

The liquid compositions of the present invention thermally harden in a time ranging from 1 to 48 hours, at a temperature of between 40 °C and 130 °C.

However, the cross-linking of the above liquid compositions to produce organic glasses with a high refractive index, generally takes place in two distinct steps.

During the first step, the composition to be cross-linked is heated in the mould having the shape of the end-product to be obtained to a temperature which is several tens of degrees lower than that which corresponds to a half life of the radicalic initiator of 10 hours. The composition is kept at this temperature for a time ranging from 6 to 15 hours.

During the second step, the composition to be cross-linked is kept at a temperature of 30 °C - 50 °C higher than that of the first step, for a time ranging from 1 to 6 hours.

The cross-linking of the above compositions is delayed by the presence of oxygen and consequently a correct hardening of the formulations requires the absence of air.

Unlike the known art, the liquid compositions of the present invention have the following advantages:

- they contain a high proportion of aromatic nuclei owing to the particular type of urethanic compound having formula (I) or (II), based on the compositions;
- all their components have well-defined structures in which there are no by-products of the polymeric type, as often occurs in the known art;
- they have a particularly low viscosity and can however be easily modulated;
- they have a monocomponent system in the sense that it is not necessary to combine two different mixtures shortly before use as often occurs for the compositions of the known art (JP 60-249101);
- in the synthesis of the urethanic compounds having formula (I) or (II), an isocyanate having formula (III) is used which is characterized by its low degree of toxicity and, in addition, compounds are used which can be easily found on the market and which are quite inexpensive;
- finally, by the cross-linking of the compositions of the present invention, organic glasses with a high refractive index are obtained which are characterized by good transparency, hardness and good processability.

The following illustrative examples provide a better understanding of the present invention and enable its embodiment but do not limit it in any way.

Table 1 shows the values (% by weight) of the compounds and catalysts used in examples 1 to 12, below.

Table 2 shows the data relating to the characteristics of the glasses obtained operating according to examples 1 to 12 below.

The $n_D^{20}$ refractive index and the value of z corresponding to the dispersion factor, are determined with an Abbe refractometer (ASTM D524).

The light transmission is determined with a Gardner Hazegard XL211 (ASTM D1003).

The Rockwell hardness (M) is measured with a Rockwell durometer (ASTM D785).

EXAMPLE 1

100.5 g (0.5 moles) of dimethyl-m-isopropenylbenzyl-isocyanate (TMI) and 25 mg (150 ppm) of hydroquinone monomethylether (HQMME) are charged into a 250 ml four-necked flask, equipped with a mechanical stirrer, drip-funnel, thermometer and nitrogen valve. The mixture is heated to 60 °C and 65.5 g (0.5 moles) of hydroxyethyl-methacrylate (HEMA) to which 25 mg of tin dibutyl-dilaurate (SnDBL) have been added, are added drop-wise, under stirring.

The whole mixture is kept at a temperature of between 60 °C and 65 °C for the duration of the addition, and the stirring is continued at this temperature until the reaction has been completed (disappearance of the I.R. signal of the NCO group, band at 2260 $cm^{-1}$) which generally occurs after 2-3 hours.

A colourless, limpid liquid is obtained, having a $n_D^{20}$ refractive index equal to 1.5252 and density equal to 1.084 (mixture A).

0.6 g of catalyst t-butyl-peroxy-2-ethylhexanoate (tBPO, 0.6% by weight) are added to 100 g of mixture A, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: from 50 °C to 70 °C in 6 hours, at 70 °C for 14 hours, from 70 °C to 100 °C in 3 hours, at 100 °C for 1 hour.

EXAMPLE 2

17.6 g of diethylenglycoldimethacrylate (EGDMA) and the catalyst t-butylperoxy-2-ethylhexanoate (tBPO, 1.5% by weight) are added to 100 g of mixture A, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: from 50 °C to 70 °C in 6 hours, at 70 °C for 14 hours, from 70 °C to 100 °C in 3 hours, at 100 °C for 1 hour.

EXAMPLE 3

10 g of styrene and the catalyst t-butylperoxy-2-ethylhexanoate (tBPO, 0.6% by weight) are added to 90 g of mixture A, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: from 50 °C to 70 °C in 6 hours, at 70 °C for 14 hours, from 70 °C to 100 °C in 3 hours, at 100 °C for 1 hour.

EXAMPLE 4

30 g of styrene and the catalyst t-butylperoxy-2-ethylhexanoate (tBPO, 0.6% by weight) are added to 70 g of mixture A, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: from 50 °C to 70 °C in 6 hours, at 70 °C for 14 hours, from 70 °C to 100 °C in 3 hours, at 100 °C for 1 hour.

EXAMPLE 5

45 g of bisphenol A diethoxyylate dimethacrylate (BPEM2-T of DKS International) and the catalyst t-butylperoxy-2-ethylhexanoate (tBPO, 0.6% by weight) are added to 34 g of mixture A, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: at 45 °C for 2 hours, at 60 °C for 15 hours, at 70 °C for 5 hours and at 80 °C for 3 hours.

EXAMPLE 6

100.5 g (0.5 moles) of dimethyl-m-isopropenyl-benzyl-isocyanate (TMI) and 25 mg (150 ppm) of hydroquinone monomethylether (HQMME) are charged into a 250 ml four-necked flask, equipped with a mechanical stirrer, drip-funnel, thermometer and nitrogen valve. The mixture is heated to 60 °C and 67.1 g (0.5 moles) of cinnamic alcohol (ACINN)to which 25 mg of tin dibutyl-dilaurate (SnDBL) have been added, are added drop-wise, under stirring.

The whole mixture is kept at a temperature of between 60 °C and 65 °C for the duration of the addition, and the stirring is continued at this temperature until the reaction has been completed (disappearance of the I.R. signal of the NCO group, band at 2260 $cm^{-1}$) which generally occurs after 2-3 hours.

A transparent liquid is obtained, having a $n_D^{20}$ refractive index equal to 1.5785 (mixture B).

10 g of hexandiol-diacrylate (EDDA), 20 g of benzyl-methacrylate (BzMA) and the catalyst t-butyl-peroxy-2-ethylhexanoate(tBPO, 2.5% by weight) are added to 70 g of mixture B, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: from 50°C to 70°C in 6 hours, at 70°C for 14 hours, from 70°C to 100°C in 3 hours, at 100°C for 1 hour.

EXAMPLE 7

100.5 g (0.5 moles) of dimethyl-m-isopropenyl-benzyl-isocyanate (TMI) and 25 mg (150 ppm) of hydroquinone monomethylether (HQMME) are charged into a 250 ml four-necked flask, equipped with a mechanical stirrer, drip-funnel, thermometer and nitrogen valve. The mixture is heated to 60°C and 33.5 g (0.25 moles) of cinnamic alcohol (ACINN) and 32.5 g (0.25 moles) of hydroxyethyl-methacrylate (HEMA) to which 25 mg of tin dibutyl-dilaurate (SnDBL) have been added, are added drop-wise, under stirring.

The whole mixture is kept at a temperature of between 60°C and 65°C for the duration of the addition, and the stirring is continued at this temperature until the reaction has been completed (disappearance of the I.R. signal of the NCO group, band at 2260 $cm^{-1}$) which generally occurs after 2-3 hours.

A limpid, homogeneous liquid is obtained, having a $n_D^{20}$ refractive index equal to 1.5552 (mixture C).

8.5 g of styrene, 11 g of benzyl-methacrylate (BzMA) and the catalyst t-butyl-peroxy-2-ethylhexanoate (tBPO, 2.5% by weight) are added to 80 g of mixture C, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: from 60°C to 70°C in 5 hours, at 70°C for 14 hours, from 70°C to 100°C in 3 hours, at 100°C for 1 hour.

EXAMPLE 8

35 g of mixture A and 35 g of mixture B cooled to room temperature, 20 g of styrene, 10 g of benzyl-methacrylate (BzMA) and the catalyst t-butyl-peroxy-2-ethylhexanoate (tBPO, 0.6% by weight) are mixed together.

The mixture is then degassed, transferred to a suitable mould composed of two pieces of glass separated by a plasticized polyvinylchloride (PVC) seal having a thickness of 2 mm and cross-linked according to the following heating cycle: from 50°C to 70°C in 6 hours, at 70°C for 14 hours, from 70°C to 100°C in 3 hours, at 100°C for 1 hour.

EXAMPLE 9

201 g (1 mole) of dimethyl-m-isopropenyl-benzyl-isocyanate (TMI), 25 mg (150 ppm) of hydroquinone monomethylether (HQMME) and 21.3 g (0.175 moles) of thiodiethylenglcyol (TDG) are charged into a 250 ml four-necked flask, equipped with a mechanical stirrer, drip-funnel, thermometer and nitrogen valve. The mixture is heated to 60°C and 84.5 g (0.65 moles) of hydroxyethyl-methacrylate (HEMA) to which 25 mg of tin dibutyl-dilaurate (SnDBL) have been added, are added drop-wise, under stirring.

The whole mixture is kept at a temperature of between 60°C and 65°C for the duration of the addition, and the stirring is continued at this temperature until the reaction has been completed (disappearance of the I.R. signal of the NCO group, band at 2260 $cm^{-1}$) which generally occurs after 2-3 hours.

A limpid, homogeneous liquid is obtained, having a $n_D^{20}$ refractive index equal to 1.5357 (mixture D).

13.5 g of styrene and the catalyst t-butyl-peroxy-2-ethylhexanoate (tBPO, 0.6% by weight) are added to 86.5 g of mixture D, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: at 60°C for 16 hours, at 70°C for 7.5 hours, from 70°C to 100°C in 1 hour, at 100°C for 1 hour.

EXAMPLE 10

25 g of bisphenol A diethoxylate dimethacrylate (BPEM2-T of DKS International), 10 g of benzyl-methacrylate (BzMA) and the catalyst t-butyl-peroxy-2-ethylhexanoate (tBPO, 0.6% by weight) are added to

25 g of mixture B obtained as described in example 6, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: at 50 °C for 3 hours, at 60 °C for 15 hours, at 70 °C for 3 hours, at 80 °C for 3 hours, at 90 °C for 1 hour and at 100 °C for 1 hour.

EXAMPLE 11

200 g (0.99 moles) of dimethyl-m-isopropenyl-benzyl-isocyanate (TMI), 71 g (0.225 moles) of bisphenol A diethoxylate (BPHE) and 2 g of hydroquinone monomethylether (HQMME) and are charged into a one to three-necked flask, equipped with a mechanical stirrer, drip-funnel, thermometer and nitrogen valve. The mixture is heated to 50 °C and 30 mg of tin dibutyl-dilaurate (SnDBL) are added under stirring.

The whole mixture is kept under stirring, at a temperature of between 65 °C and 75 °C, until the bisphenol A diethoxylate has completely dissolved. A further 101.5 g (0.5 moles) of dimethyl-m-isopropenyl-benzyl-isocyanate (TMI) are added and the temperature is brought to 60 °C. 136.5 g (1.05 moles) of hydroxyethyl-methacrylate (HEMA) are then added drop-wise.

The whole mixture is kept at a temperature of between 60 °C and 65 °C for the duration of the addition, and the stirring is continued at this temperature until the reaction has been completed (disappearance of the I.R. signal of the NCO group, band at 2260 $cm^{-1}$) which generally occurs after 2-3 hours.

A limpid, colourless liquid is obtained, having a $n_D^{20}$ refractive index equal to 1.5420 (mixture E).

4 g of styrene, 2 g of benzyl-methacrylate (BzMA) and the catalyst t-butyl-peroxy-2-ethylhexanoate (tBPO, 0.64% by weight) are added to 19.4 g of mixture E, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: at 65 °C for 18 hours, at 75 °C for 7 hours, at 85 °C for 16 hours, at 95 °C for 1 hour, at 105 °C for 2 hours.

EXAMPLE 12

5.4 g of styrene and the catalyst t-butyl-peroxy-2-ethylhexanoate (tBPO, 0.6% by weight) are added to 21.6 g of mixture E, cooled to room temperature.

The mixture is then degassed, transferred to a suitable mould composed of two pieces of glass separated by a plasticized polyvinylchloride (PVC) seal having a thickness of 2 mm and cross-linked according to the following heating cycle: at 60 °C for 16 hours, at 75 °C for 8 hours, at 85 °C for 16 hours, at 90 °C for 3 hours and at 100 °C for 1 hour.

TABLE 1

| Example N° | Component % w/w | Catalyst (tBPO) % w/w |
|---|---|---|
| 1 | (TMI) / (HEMA) : 100 | 0.6 |
| 2 | (TMI) / (HEMA) : 85 + (EGDMA) : 15 | 1.5 |
| 3 | (TMI) / (HEMA) : 90 + STYRENE : 10 | 0.6 |
| 4 | (TMI) / (HEMA) : 70 + STYRENE : 30 | 0.6 |
| 5 | (TMI) / (HEMA) : 43 + (BPEM2-T): 57 | 0.6 |
| 6 | (TMI) / (ACINN) : 70 + (EDDA) : 10 + (BzMA) : 20 | 2.5 |
| 7 | (TMI) / (HEMA) : 40 + (TMI) / (ACINN) : 40 + (BzMA) : 11.5 + (STYRENE) : 8.5 | 1 |
| 8 | (TMI) / (HEMA) : 35 + (TMI) / (ACINN) : 35 + (BzMA) : 10 + (STYRENE) : 20 | 0.6 |
| 9 | (TMI) / (HEMA) : 60.5 + 2 (TMI) / $(CH_2CH_2)_2S$ : 26 + STYRENE : 13.5 | 0.6 |
| 10 | (TMI) / (ACINN) : 41.6 + (BPEM2-T) : 41.6 + (BzMA) : 16.8 | 0.6 |
| 11 | (TMI) / (HEMA) : 53.5 + (TMI) / (BPHE) : 22.8 + (BzMA) : 8 + STYRENE : 15.7 | 0.64 |
| 12 | (TMI) / (HEMA) : 56 + (TMI) / (BPHE) : 24 + STYRENE : 20 | 0.6 |

TABLE 2

| Example N° | $n_D^{20}$ | z | Light transmission | Rockwell hardness |
|---|---|---|---|---|
| 1 | 1.5545 | 37.5 | 91.5 | 118.0 |
| 2 | 1.5605 | 37.0 | 90.8 | 120.0 |
| 3 | 1.5580 | 37.0 | 91.4 | 120.0 |
| 4 | 1.5645 | 36.0 | 91.1 | 119.0 |
| 5 | 1.5600 | 37.0 | 91.1 | 106.0 |
| 6 | 1.5845 | 35.0 | 90.5 | 80.0 |
| 7 | 1.5785 | -- | 90.5 | -- |
| 8 | 1.5800 | 35.0 | 90.8 | 118.0 |
| 9 | 1.5678 | 37.0 | -- | -- |
| 10 | 1.5820 | 35.5 | 90.7 | 110.0 |
| 11 | 1.5690 | 36.5 | 90.9 | 123.0 |
| 12 | 1.5708 | 36.5 | 91.0 | 122.5 |

## Claims

**1.** Liquid compositions which can be polymerized thermally or via radicals, basically composed of urethanic compounds containing unsaturations of the styrenic or (meth)acrylic type, selected from those having the following general formulae (I) or (II):

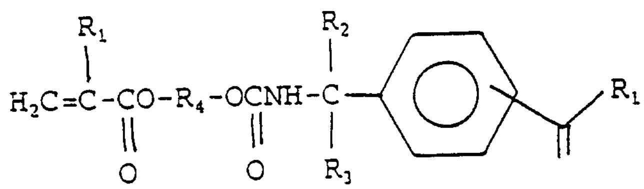

(I)

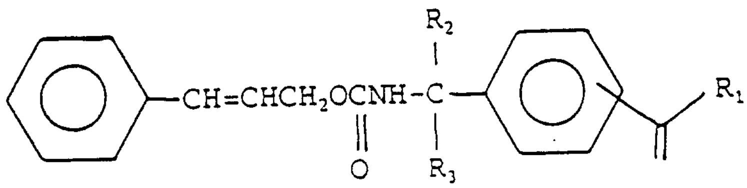

(II)

wherein:

- $R_1$, $R_2$ and $R_3$, the same or different, represent a hydrogen atom or a methyl;
- $R_4$ represents a $C_2$-$C_{10}$ (iso)alkylic residue of at least one diol.

**2.** Liquid compositions according to claim 1, wherein the urethanic compounds having general formula (I) or (II) are those wherein $R_4$ represents an alkyl residue of a diol having one of the following formulae: $-CH_2CH_2-$; $-CH_2CHCH_3$;

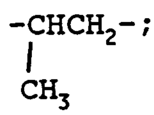

$-CH_2CH_2CH_2CH_2-$.

**3.** Liquid compositions according to claims 1 or 2, wherein the urethanic compounds having general formula (I) or (II) can be used alone or mixed with each other or can be mixed with one or more reactive diluents of the acrylate and/or methacrylate and/or styrenic type, or mixed with reactive compounds containing unsaturated groups.

**4.** Liquid compositions according to claim 3, wherein the reactive diluents of the acrylate and/or methacrylate and/or styrenic type can be mono-, bi- or poly-functional.

**5.** Liquid compositions according to claim 4, wherein the reactive diluents are selected from those belonging to the groups of acrylic, methacrylic and styrenic compounds having a high refractive index, low viscosity and good optical properties.

**6.** Liquid compositions according to claims 4 or 5, wherein the reactive diluents of the acrylate and/or methacrylate type are: hexandiol-diacrylate, benzyl-methacrylate, phenyl-methacrylate, phenoxyethyl-methacrylate, cyclohexyl-methacrylate; isobornyl-methacrylate; 2-naphthyl-methacrylate; (o, m, p)-phenylene-dimethacrylate; methylthio-methacrylate; thiodiethylen-dimethacrylate; diethylenglycol-dimethacrylate; and the corresponding acrylates.

**7.** Liquid compositions according to claims 4 or 5, wherein the reactive diluents of the styrenic type, are: m-divinylbenzene, m-ethylvinylbenzene, styrene, α-methylstyrene, (o, m, p)-chlorostyrene, (o, m, p)-bromostyrene, 2-isopropenyl-naphthalene.

**8.** Liquid compositions according to any of the previous claims from 3 to 7, wherein the quantity of reactive diluents of the acrylate and/or methacrylate and/or styrenic type is such that the weight ratio urethanic compounds/reactive diluent is between 99:1 and 45:55.

**9.** Liquid compositions according to claim 3 wherein the reactive compounds containing unsaturated groups, can be either mono-, bi- or poly-functional.

**10.** Liquid compositions according to claim 9, wherein the reactive compounds containing unsaturated groups are:

a) styrene-urethanic resins having the following structural formula:

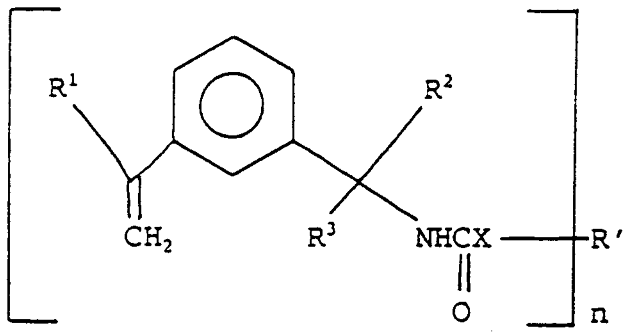

wherein:

- R' represents an alkyl or cyclo-alkyl residue of a glycol, polyol, polythiol, dimercaptan, containing from 2 to 19 carbon atoms and 1 or 2 sulphur or oxygen atoms;
- $R^1$, $R^2$ and $R^3$, the same or different, represent a hydrogen atom or a methyl;
- X represents an oxygen or sulphur atom;
- n is 2 or 3;

b) bis(alkyleneoxyphenyl)diacrylates or dimethacrylates, having the following general formula:

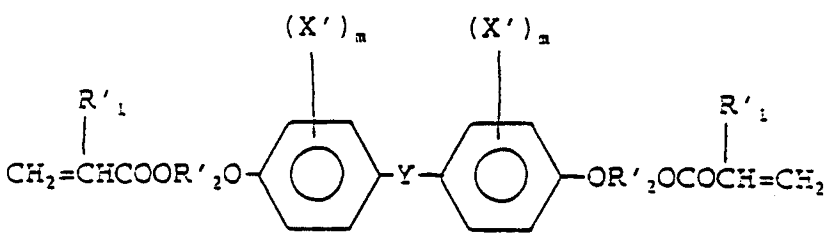

wherein:

- $R'_1$ represents a hydrogen atom or a methyl;
- $R'_2$ represents one of the following groups: $-CH_2CH_2-$;

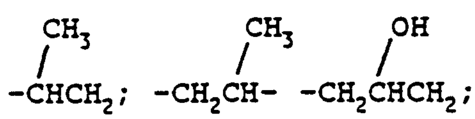

- Y represents an oxygen atom or one of the following groups: $-SO_2-$; $-CH_2-$;

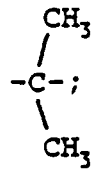

- X' represents a chlorine or bromine atom;
- m is 0, 1 or 2.

**11.** Liquid compositions according to any of the previous claims, including radicalic polymerization initiators, stabilizers, bleaching agents, detaching agents, dyes, UV absorbers.

**12.** Procedure for the preparation of the urethanic compounds having formula (I) or (II) which includes the reaction of a mole of an isocyanate having formula (III):

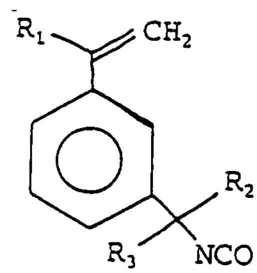

wherein $R_1$, $R_2$ and $R_3$ have the meaning defined above, with a mole of a hydroxyalkyl(meth)acrylate and/or cinnamic alcohol corresponding to structural formulae (IV) and (V):

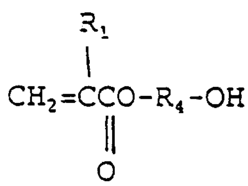 (IV) 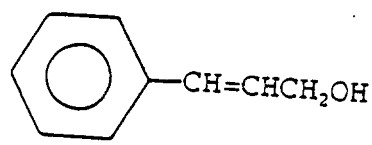 (V)

wherein $R_1$ and $R_4$ have the meaning specified above.

**13.** Procedure according to claim 12, wherein the reaction generally takes place in a time ranging from 2 to 24 hours, at a temperature of between 20 °C and 120 °C, adding hydroxyalkyl(meth)acrylate having formula (IV) or cinnamic alcohol having formula (V) to the isocyanate having formula (III) or viceversa.

**14.** Organic glasses with a high refractive index obtained by the radicalic cross-linking of the liquid compositions claimed in points 1 to 11 in the presence of 0.01% - 5% by weight of a peroxide or 0.001% - 0.5% by weight of an azoderivative.